(19) Europäisches Patentamt
European Patent Office
Office européen des brevets

(11) Publication number: 0 084 236
A2

# (12) EUROPEAN PATENT APPLICATION

(21) Application number: 82306664.2

(22) Date of filing: 14.12.82

(51) Int. Cl.³: C 07 D 233/56
C 07 D 249/08, A 61 K 31/415
A 61 K 31/41

(30) Priority: 22.12.81 GB 8138663

(43) Date of publication of application:
27.07.83 Bulletin 83/30

(84) Designated Contracting States:
AT BE CH DE FR GB IT LI LU NL SE

(71) Applicant: FBC LIMITED
Hauxton
Cambridge CB2 5HU(GB)

(72) Inventor: Wells, Wilfred Hase
23 Welbeck Road
Radcliffe-on-Trent Nottingham NG12 1DH(GB)

(74) Representative: Waldman, Ralph David et al,
Industrial Property Department FBC Limited
Chesterford Park Research Station
Saffron Walden, Essex CB10 1XL(GB)

## (54) Fungicidal heterocyclic compounds and compositions containing them.

(57) Compounds having fungicidal activity are of formula 1

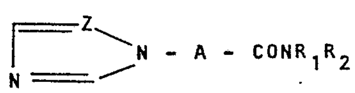

where Z is CH or N, A is an optionally branched alkylene group which may be substituted and $R_1$ and $R_2$, which may be the same or different, are optionally substituted alkyl, cycloalkyl, alkenyl, or alkynyl, together with acid addition salts and complexes of these compounds with metal salts.

EP 0 084 236 A2

Croydon Printing Company Ltd.

CASE BA 81/38663 0084236

# FUNGICIDAL HETEROCYCLIC COMPOUNDS AND COMPOSITIONS CONTAINING THEM

This invention relates to new compounds having fungicidal activity.

In British Patent No. 1469772, there are claimed compounds having fungicidal activity of the formula

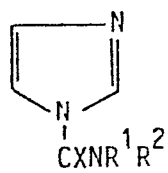

in which X is oxygen or sulphur, $R^1$ is optionally substituted alkyl, alkenyl, alkynyl, cycloalkyl, phenyl, phenylalkyl, phenylalkenyl, phenoxyalkyl or phenylthioalkyl and $R^2$ is optionally substituted phenyl, phenylalkyl, phenylalkenyl, phenoxyalkyl or phenylthioalkyl, provided that when $R^1$ is methyl or phenyl, $R^2$ is substituted phenyl or optionally substituted phenylalkyl, phenylalkenyl, phenoxyalkyl or phenylthioalkyl. In these compounds the carbonyl or thiocarbonyl group is directly attached to the nitrogen of a heterocyclic group.

According to the present invention there are provided compounds of formula I

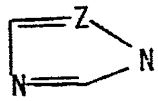 - A - $CONR_1R_2$ I

where Z is CH or N, A is an optionally branched alkylene group which may be substituted and $R_1$ and $R_2$, which may be the same or different are optionally substituted alkyl, cycloalkyl, alkenyl or alkynyl together with acid addition salts and complexes of these compounds with metal salts.

The complexes are usually with a salt of formula $MA_2$ in which M is a divalent metal cation, e.g. copper, calcium or preferably manganese, and A is an anion, e.g. chloride, nitrate or a hydrocarbon sulphonate, e.g. dodecylbenzenesulphonate. The molar ratio of the compound to metal salt is usually 2 or 4 to 1. Acid addition salts are usually formed with strong inorganic or organic acids, e.g. hydrochloric acid or oxalic acid.

The alkyl groups are preferably of 1 to 4 carbon atoms and may be branched. When substituted, preferred substituents are optionally substituted phenyl, heteroaryl, optionally substituted phenoxy, cycloalkyl, optionally substituted (e.g. by alkyl), cyano, halo, alkoxy, optionally substituted (e.g. by alkoxy), alkylthio, hydroxy or alkoxycarbonyl. The alkylene

group is generally of 1 to 3 carbon atoms and is preferably methylene. Optional substituents include phenyl and alkoxycarbonyl. Phenyl or phenoxy groups may be substituted by one or more groups, which may be the same or different, e.g. alkyl, haloalkyl, alkoxy, hydroxy, halo, phenoxy, alkylsulphonyloxy, optionally substituted (e.g. by halogen) alkoxycarbonyl or substituted amino, e.g. dialkylamino, arylalkylamino or alkanoylamino. Heteroaryl groups include thienyl, pyridyl and furyl.

A particularly preferred group of compounds are those in which one of $R^1$ and $R^2$ is substituted phenoxyalkyl and the other is cycloalkyl, alkenyl, alkynyl, alkyl substituted by alkoxycarbonyl or hydroxy or especially unsubstituted alkyl.

These compounds have fungicidal activity against a range of fungi which attack agricultural crops. For example they can be used to control powdery mildew (*Erysiphe graminis*) and glume blotch (*Septoria nodorum*) on crops such as wheat, barley and oats and other fungal diseases of cereal crops such as leaf spot (*Pyrenophora avenae*) on oats, rusts such as wheat brown rust (*Puccinia recondita*), leaf stripe (*Pyrenophora graminea*) on barley, snow mould (*Fusarium nivale*) on rye and blast (*Pyricularia oryzae*) on rice. Many horticultural crops can also be treated with the compounds of the invention for example in the protection of fruit crops against *Botrytis ci nerea*, powdery mildew (*Podosphaera leucotricha*) and scab (*Venturia*

anaequalis) on apple tress, powdery mildew (Sphaerotheca pannosa) on roses, powdery mildew (Sphaerotheca fuliginea) on cucurbits and various fungal diseases on legumes such as beans, soybeans and peanuts. Treatment can be by means of direct application to the crop or, in countering seed borne diseases, by seed treatment.

In addition to their uses in the treatment of agricultural crops the compounds find many applications as general fungicides in, for example, the industrial sphere where materials such as adhesives, starch, pastes, insulation, oils and paper products often need protection against attack by fungi.

The invention thus also provides a method of combating fungi at a locus infested or liable to be infested therewith, which comprises applying to the locus a compound of formula I.

The invention also provides an agricultural composition comprising a compound of formula I in admixture with an agronomically acceptable diluent or carrier.

The composition of the invention may of course include more than one compound of the invention

In addition the composition can comprise one or more additional active ingredients, for example compounds known to possess plant-growth regulant, herbicidal, fungicidal, insecticidal or acaricidal properties. Alternatively the compounds of the invention can be used in sequence with the

other active ingredient. Fungicides which can be used in conjunction with the compounds of the present invention include maneb, zineb, mancozeb, thiram, ditalimfos, tridemorph, fenpropemorph, imazalil, propiconazole, triadimefon, triadimenol, diclobutrazol, fluotrimazole, ethirimol,fenarimol, nuarimol, triforine, pyrocarbolid, tolclofos-methyl, oxycarboxin, carbendazim, benomyl, thiophanate, thiophanate-methyl, thiabendazole, propineb,metalaxyl, dicloran, dithianon, fuberidazole, dodine, chlorothalonil, cyprofuram, dichlofluanid, sulphur, copper compounds, iprodione, ziram, nabam, prochloraz (and metal complexes of this e.g. the manganese chloride complex), zineb-ethylene thiuramsulphide adduct, captan, captafol, benodanil, mepronil, carboxin, guazatine, validamycin, vinclozolin, tricyclazole, quintozene, pyrazophos, furmecyclox, propamocarb, procymidone, kasugamycin, furalaxyl, folpet, fenfuram, ofurace, etridiazole, aluminium tris (ethyl phosphonate) and benalaxyl.

The diluent or carrier in the composition of the invention can be a solid or a liquid optionally in association with a surface-active agent, for example a dispersing agent, emulsifying agent or wetting agent. Suitable surface-active agents include anionic compounds such as a carboxylate, for example a metal carboxylate of a long chain fatty acid; an N-acylsarcosinate; mono- or di-esters of phosphoric acid with fatty alcohol ethoxylates or salts of such esters; fatty

alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl-aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonatedphenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate of dioctyl succinate. Nonionic agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene oxide, acetylenic glycols such as 2,4,7,9-tetramethyl-5-decyn-4,7-diol, or ethoxylated acetylenic glycols. Examples of a cationic surface-active agent include, for instance, an aliphatic mono-, di-, or polyamine as an acetate, naphthenate or oleate; an oxygen-containing amine such as an amine oxide or polyoxyethylene alkylamine; an amide-linked amine prepared by the condensation of a carboxylic acid with a di- or polyamine;

or a quaternary ammonium salt.

The composition of the invention can take any form known in the art for the formulation of fungicidal and similar compounds, for example, a solution, a dispersion, an aqueous emulsion, a dusting powder, a seed dressing, a fumigant, a smoke, a dispersible powder, an emulsifiable concentrate or granules. Moreover it can be in a suitable form for direct application or as a concentrate or primary composition which requires dilution with a suitable quantity of water or other diluent before application.

As a dispersion, the composition comprises a compound of the invention dispersed in a liquid medium, preferably water. It is often convenient to supply the consumer with a primary composition which can be diluted with water to form a dispersion having the desired concentration. The primary composition can be provided in any one of the following forms. It can be a dispersible solution which comprises a compound of the invention dissolved in a water-miscible solvent with the addition of a dispersing agent. A further alternative comprises a compound of the invention in the form of a finely ground powder in association with a dispersing agent and intimately mixed with water to give a paste or cream which can if desired be added to an emulsion of oil in water to give a dispersion of active ingredient in an aqueous oil emulsion.

An emulsion comprises a compound of the invention dissolved in a water-immiscible solvent which is formed into an emulsion with water in the presence of an emulsifying agent. An emulsion of the desired concentration can be formed from a primary composition of the following types. A concentrated stock emulsion can be supplied comprising a compound of the invention in combination with an emulsifying agent, water and a water-immiscible solvent. Alternatively an emulsifiable concentrate can be supplied to the user comprising a solution of a compound of the invention in a water-immiscible solvent containing an emulsifying agent.

A dusting powder comprises a compound of the invention intimately mixed and ground with a soil pulverulent diluent, for example, kaolin.

A granular solid comprises a compound of the invention associated with similar diluents to those which may be employed in dusting powders, but the mixture is granulated by known methods. Alternatively it comprises the active ingredient adsorbed or absorbed on a pre-granular diluent, for example, Fuller's earth, attapulgite or limestone grit.

The concentration of the active ingredient in the composition of the present invention, is applied to plants is preferably within the range of 0.001 to 3.0 per cent by weight, especially 0.001 to 1.0 per cent by weight. In a primary composition the amount of active ingredient can vary widely and

can be, for example, from 5 to 95 per cent by weight of the composition.

In the method of the invention the compound is generally applied to seeds, plants or their habitat. Thus, the compound can be applied directly to the soil before, at or after drilling so that the presence of active compound in the soil can control the growth of fungi which may attack seeds. When the soil is treated directly the active compound can be applied in any manner which allows it to be intimately mixed with the soil such as by spraying, by broadcasting a solid in the form of granules, or by applying the active ingredient at the same time as drilling by inserting it in the same drill as the seeds. A suitable application rate is within the range of from 0.05 to 20 kg per hectare, more preferably from 0.1 to 10 kg per hectare.

Alternatively the active compound can be applied directly to the plant by, for example, spraying or dusting either at the time when the fungus has begun to appear on the plant or before the appearance of fungus as a protective measure. In both such cases the preferred mode of application is by foliar spraying. It is generally important to obtain good control of fungi in the early stages of plant growth as this is the time when the plant can be most severely damaged. For cereal crops such as wheat, barley and oats it is often desirable to spray the plant at or before growth stage 5 although additional treatments by

spraying when the plant is more mature can augment resistance to the growth or spread of fungi. The spray or dust can conveniently contain a pre- or post-emergence herbicide if this is thought necessary. Sometimes, it is practicable to treat the roots of a plant before or during planting, for example, by dipping the roots in a suitable liquid or solid composition.

When the active compound is applied directly to the plant a suitable rate of application is from 0.01 to 10 kg. per hectare, preferably from 0.05 to 5 kg. per hectare.

When the compounds of the invention are used as growth regulators they are usually best applied during the vegetative stages of growth at similar rates as above. In the case of crops such as legumes, cotton and sunflowers, application preferably takes place during the late vegetative stage of growth, just prior to or just after the onset of flowering. In the case of pot plants and turf, earlier application, during the early vegetative stage of growth, is more appropriate.

The compounds of the invention may be prepared by reacting imidazole or 1,2,4-triazole or their sodium salt with a compound of formula II

$$X\text{-}A\text{-}CONR_1R_2 \qquad \text{II}$$

where X is halogen, preferably chlorine. This reaction is usually carried out at a temperature of 50 to 150°C and

generally under reflux. A solvent is generally used, e.g. an ether such as tetrahydrofuran. It is generally preferred to use the sodium salt of imidazole or triazole. This is usually prepared *in situ* using sodium hydride. If the sodium salt is not used it is generally necessary to use an excess of imidazole or triazole.

The compounds of formula II may be prepared by the reaction of a carbonyl halide of formula III

X-A-COX III

with an amine of formula $NHR_1R_2$. Some of the compounds of formula II are new compounds and a further aspect of the present invention includes these new compounds.

Some of the amines may be new in which case they can be prepared in conventional manner, e.g. by reaction of $R_1NH_2$ with $R_2X$, where X is as defined above.

The invention is illustrated in the following Examples. Structures of isolated novel compounds were confirmed by elemental and/or other appropriate analysis.

## Example 1

Triethylamine (5.7g) was added to a stirred solution of *N*-[2-(2,4,6-trichlorophenoxy)ethyl]propylamine (16.125g) in

toluene (80mls). The mixture was cooled in ice and chloroacetyl chloride (6.4g) in toluene (20mls) added dropwise over a period of 15 minutes. Stirring was continued for a further hour and the reaction mixture was filtered to remove the triethylamine salt and the filtrate was washed with dilute hydrochloric acid and water and then dried over anhydrous magnesium sulphate. After removal of the solvent an oil remained. The oil was triturated with light petroleum and the resulting product dried to give 2-chloro-N-propyl-N-[2-(2,4,6-trichlorophenoxy)ethyl]acetamide, m.p. 69-70°C. (Intermediate A).

A solution of imidazole (1.55g) in dry tetrahydrofuran (50ml) was added dropwise to a stirred suspension of sodium hydride (1g of a 50%w/w dispersion in oil) in dry tetrahydrofuran (50ml). Stirring was continued at ambient temperature for one hour and then under reflux for a further hour. Intermediate A (7.18g) dissolved in dry tetrahydrofuran (100mls) was added dropwise over a period of 30 minutes and the reaction mixture was heated under reflux for a further 22 hours. The solvent was removed under vacuum and a brown oil isolated which was triturated under light petroleum (boiling range 40-60°C), and ether and dissolved in dichloromethane. The solution was washed twice with water and dried over magnesium sulphate. The solvent was removed under vacuum to yield a thick viscous oil which solidified on standing. The

product was recrystallised from petroleum (boiling range 80-100°C) to give N-propyl-N-[2-(2,4,6-trichlorophenoxy)-ethyl]-2-imidazol-1-ylacetamide, m.p. 64-9°C.

Example 2

In a similar manner to Example 1, the following products were obtained. Where no physical data are given for the intermediate, this was used in the next stage without purification. In the case of compound 39, imidazole was reacted in the form of the free base using twice the molar rate.

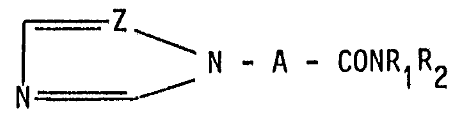

| Compound Number | Z | A | $R_1$ | $R_2$ | Physical Constant (°C) | Intermediate Physical Constant (°C) |
|---|---|---|---|---|---|---|
| 2 | N | $CH_2$ | Pr | 2,4,6-$Cl_3$-PhO $(CH_2)_2$- | oil | 69-70 |
| 3 | CH | $CH_2$ | Pr | PhO$(CH_2)_2$- | 52-54 | oil |
| 4 | CH | $CH_2$ | Pr | PhO$(CH_2)_4$- | oil | |
| 5 | N | $CH_2$ | Pr | $Pr^i$ | bp 138-40/0.2mm | bp 76-8/0.2mm |
| 6 | N | $CH_2$ | Pr | PhO$(CH_2)_2$- | 68-70 | oil |
| 7 | N | -CH$(CH_3)$- | Pr | PhO$(CH_2)_2$- | oil | oil |
| 8 | N | $CH_2$ | Et | 2,4-$Cl_2$-Ph- | 122-4 | 65-67 |
| 9 | CH | $(CH_2)_2$ | Bu | 2,4,6-$Cl_3$-PhO$(CH_2)_2$- | oil | oil |
| 10 | N | $CH_2$ | Pr | 2,4,-$Cl_2$,6-Me-Ph-O$CH_2$CH$(CH_3)$- | oil | oil |
| 11 | CH | $CH_2$ | Pr | 2-thenyl | 75-7 | |
| 12 | N | $CH_2$ | Pr | Et | oil | bp 90-2/0.1mm |
| 13 | CH | $CH_2$ | Pr | 2Cl-PhO$(CH_2)_2$- | 131-2 | bp 166-172/0.5mm |

| Compound Number | Z | A | $R_1$ | $R_2$ | Physical Constant (°C) | Intermediate Physical Constant (°C) |
|---|---|---|---|---|---|---|
| 14 | CH | $CH_2$ | Pr | 4-Cl-PhO$(CH_2)_2$- | 132-4 | |
| 15 | CH | $CH_2$ | Pr | 2,6-$Cl_2$-PhO$(CH_2)_2$- | 80-2 | |
| 16 | CH | $CH_2$ | Pr | 2Me-PhO$(CH_2)_2$- | 109-111 | |
| 17 | CH | $CH_2$ | $Pr^i$ | 2,4,6-$Cl_3$-PhO$(CH_2)_2$- | 89-91 | |
| 18 | CH | $CH_2$ | $PhCH_2$ | 2,4,6-$Cl_3$-PhO$(CH_2)_2$- | oil | oil |
| 19 | CH | $CH_2$ | Et | 2,4,6-$Cl_3$-PhO$(CH_2)_2$- | oil | oil |
| 20 | CH | $CH_2$ | Pr | 4-$Bu^t$-PhO-$(CH_2)_2$- | gum | |
| 21 | CH | $CH_2$ | Pr | 2,4,6-$Cl_3$-PhO$(CH_2)_3$- | 94-5 | |
| 22 | CH | $CH_2$ | $-CH_2CH=CH_2$ | 2,4,6-$Cl_3$-PhO$(CH_2)_2$- | oil | oil |
| 23 | CH | $CH_2$ | 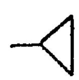 | 2,4,6-$Cl_3$-PhO$(CH_2)_2$- | 141-3 | |
| 24 | CH | $CH_2$ | $-CH_2C\equiv CH$ | 2,4,6-$Cl_3$-PhO$(CH_2)_2$- | gum | |

| Compound Number | Z | A | $R_1$ | $R_2$ | Physical Constant (°C) | Intermediate Physical Constant (°C) |
|---|---|---|---|---|---|---|
| 25 | CH | $CH_2$ | $Pr^i$ | $4-Bu^t-PhO-(CH_2)_2-$ | gum | |
| 26 | CH | $CH_2$ | $CH_3$<br>\\<br>-CH-COOEt | $2,4,6-Cl_3-PhO(CH_2)_2-$ | 89 | oil |
| 27 | CH | $CH_2$ | $Pr^i$ | $4-Br-PhO(CH_2)_2)-$ | 30 | |
| 28 | CH | $CH_2$ | $-CH_2CH_2-$<br>OMe | $2,4,6-Cl_3-PhO(CH_2)_2-$ | oil | |
| 29 | CH | $CH_2$ | $-CH_2CH_2-$<br>OH | $2,4,6-Cl_3-PhO(CH_2)_2-$ | oil | |
| 30 | CH | -CH-<br>\|<br>Ph | Pr | $2,4,6-Cl_3-PhO(CH_2)_2-$ | oil | |
| 31 | CH | $(CH_2)_3$ | Pr | $2,4,6-Cl_3-PhO(CH_2)_2-$ | oil | |
| 32 | CH | -CH-<br>/<br>COOEt | $Pr^i$ | $2,4,6-Cl_3-PhO(CH_2)_2-$ | oil | |
| 33 | CH | $CH_2$ | Pr | $3-CF_3-PhO(CH_2)_2-$ | | 83-5 |
| 34 | CH | $CH_2$ | Pr | $4-EtOCO-PhO(CH_2)_2-$ | | 83-5 |
| 35 | CH | $CH_2$ | Pr | $2,6-Me_2-PhO(CH_2)_2-$ | oil | |

| Compound Number | Z | A | $R_1$ | $R_2$ | Physical Constant (°C) | Intermediate Physical Constant (°C) |
|---|---|---|---|---|---|---|
| 36 | CH | $CH_2$ | Pr | 2-PhO-PhO$(CH_2)_2$- | 92-4 | 54-6 |
| 37 | CH | $CH_2$ | Pr | 2,4,6-$Me_3$-PhO$(CH_2)_2$- | oil | 68-70 |
| 38 | CH | $CH_2$ | Bu | 2,4,6-$Cl_3$-PhO-$(CH_2)_2$- | oil | |
| 39 | CH | $CH_2$ | Pr | 4-$CH_3$CONH-PhO-$(CH_2)_2$- | 88-90 | 133-134 |
| 40 | CH | $CH_2$ | -$CH_2CH_2$-OCOMe | 2,4,6-$Cl_3$-PhO$(CH_2)_2$- | gum | |
| 41 | CH | $CH_2$ | Pr | 4-$Bu^t$-Ph$(CH_2)_2$-CH(Me)- | 109-111 | |
| 42 | CH | $CH_2$ | Pr | 2,4-$Cl_2$-Ph-$(CH_2)_3$- | oil | |
| 43 | CH | $CH_2$ | Pr | 4-MeO-PhO-$(CH_2)_2$- | 156-8 | 100-1 |
| 44 | CH | $CH_2$ | Pr | 4-OH-PhO$(CH_2)$- | 93-4 | |
| 45 | CH | -CH(Me)- | Pr | 2,4,6-$Cl_3$-PhO-$(CH_2)_2$- | syrup | |
| 46 | CH | $CH_2$ | Pr | -CH(Me)$CH_2$O$Bu^t$ | syrup | oil |
| 47 | CH | $CH_2$ | 2-furylmethyl | 2,4,6-$Cl_3$-PhO-$(CH_2)_2$- | syrup | syrup |

Example 3

Compound 2 of Example 2 (3.9g) in ethanol 20 ml was mixed with a solution of manganous chloride tetrahydrate (1.0 gm) in water (5 ml) and the mixture kept overnight at ambient temperature. Evaporation of the solvents gave a sticky gum which was dissolved in methanol. Addition of a small volume of ether and chilling gave a solid, which was recrystallised from a methanol/ether mixture to give N-propyl-N-[2-(2,4,6-trichlorphenoxy)ethyl]-2-(imidazol-1-yl) - acetamide manganese (II) chloride complex (2:1), mp 138-142°C.

In a similar manner there was obtained the copper (II) chloride complex (2:1), mp 172-4°C.

Example 4

Compound 2 of example 2 (1.9g) and oxalic acid (0.45g) were dissolved in acetone. After a few minutes a solid formed which was collected and recrystallised from ethanol/light petroleum (bp 60-80°C) mixture to give N-propyl-N-[2-(2,4,6-trichlorophenoxy)ethyl]-2-(imidazol-1-yl)-acetamide oxalate (2:1), mp 134-6°C.

Example 5

Aqueous acetone suspensions of the compound under test, at various concentrations, containing 125g per litre of polyoxyethylene sorbitan monolaurate wetting agent, were applied to the leaves (sprayed to "run-off") of barley plants (*Hordeum vulgaris*) having 2 fully expanded leaves. The treated plants together with controls, treated with aqueous solutions of wetting agent only, were inoculated 24 hours later by spraying the leaves to "run-off" with an aqueous suspension of spores of barley powdery mildew (*Erysiphe graminis*). The plants were transferred to a controlled environment room for ten days after which the disease control was assessed.

The product of Example 1 and compounds 2, 4, 5 and 8 to 10 and 12 of Example 2 gave greater than 50% control of the disease compared with controls at a concentration of 1000ppm (w/v) or less. In a similar test in which the plants were treated after the disease was established the product of Example 1 and compounds 2, 3, 4 and 9 to 11 gave greater than 50% control of the disease at 1000ppm (w/v) or less.

In a similar test in which the soil surrounding the roots of the plants was drenched at the same time as the leaves were sprayed, compounds 13 to 47 of Example 2 and the products of

Examples 3 and 4 gave greater than 50% control of the disease at 500ppm (w/v) or less.

Example 6

Aqueous acetone suspensions of the compound under test, at various concentrations, containing 125g per litre each of polyoxyethylene sorbitan monolaurate and ethylene oxide propylene oxide block co-polymer wetting agents were applied to the leaves (sprayed to "run-off") and to the soil surrounding the roots (1ml liquid/25ml soil) of wheat plants (Triticum aestivum) having 2 fully expanded leaves. The treated plants together with controls, treated with aqueous solutions of wetting agent only, were inoculated 24 hours later by spraying the leaves to "run-off" with an aqueous suspension of the spores of wheat brown rust (Puccinia recondita). The plants were then placed in an atmosphere of 100% relative humidity for 24 hours before transferring to the controlled environment room for 12 days, after which the disease control was assessed.

Compounds 25, 26, 30, 32 and 38 of Example 2 gave greater than 50% control of the disease compared with controls at a concentration of 500ppm (w/v) or less.

Example 7

In a similar manner to the first test in Example 5, compounds 17, 22, 23 and 38 of Example 2 gave greater than 50% control of rice blast (*Pyricularia oryzae*) on rice plants (*Oryza sativa*) at a concentration of 500ppm (w/v) or less.

Example 8

The following concentrates were formulated as below:

| Emulsifiable concentrate | | % w/v |
|---|---|---|
| Compound 2 of example 2 | | 20 |
| Toximul S (blend of anionic and nonionic emulsifiers) | | 6 |
| Cyclohexanone | | 20 |
| Xylene | to | 100 |

| Wettable Powder | | w/w |
|---|---|---|
| Compound of Example 1 | | 25 |
| Silica | | 5 |
| Naphthalene sulphonic acid | | 10 |
| Fatty alcohol polyglycol ether absorbed on silica | | 3 |
| China Clay | to | 100 |

CLAIMS

1. Compounds of formula I

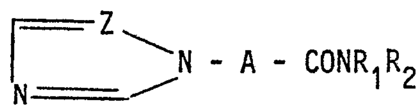

where Z is CH or N, A is an optionally branched alkylene group which may be substituted and $R_1$ and $R_2$, which may be the same or different are optionally substituted alkyl, cycloalkyl, alkenyl or alkynyl together with acid addition salts and complexes of these compounds with metal salts.

2. Compounds according to claim 1 in which the alkyl group is optionally substituted by phenyl, heteroaryl, optionally substituted phenoxy, cycloalkyl (optionally substituted by alkyl), cyano, halo, alkoxy, (optionally substituted by alkoxy), alkylthio, hydroxy or alkoxycarbonyl, the alkylene group is optionally substituted by phenyl or alkoxycarbonyl and wherein any phenyl or phenoxy group is optionally substituted by one or more groups, which may be the same or different, and are alkyl, haloalkyl, alkoxy, hydroxy, halo, phenoxy, alkylsulphonyloxy, haloalkylsulponyloxy,

alkoxycarbonyl, dialkylamino, alkanoylamino, or arylalkylamino.

3. Compounds according to claim 1 or 2 in which A is methylene.

4. Compounds according to any one of the preceding claims in which one of $R_1$ and $R_2$ is substituted phenoxyalkyl and the other is cycloalkyl, alkenyl, alkynyl, alkyl substituted by alkoxycarbenyl or hydroxy or unsubstituted alkyl.

5. Agricultural compositions comprising a compound claimed in any one of the preceding claims in admixture with an agronomically acceptable diluent or carrier.

6. A method of combating fungi at a locus infested or liable to be infested therewith, which comprises applying to the locus a compound as claimed in any one of claims 1 to 4.

7. A method of preparing a compound as claimed in claim 1 which comprises reacting imidazole or 1,2,4-triazole or their sodium salt with a compound of formula II

$$X\text{-}A\text{-}CONR_1R_2 \qquad II$$

where X is halogen, preferably chlorine.

8. Compounds of formula II

$$X\text{-}A\text{-}CONR_1\ R_2 \qquad II$$

where X is as defined in claim 7 and A, $R_1$ and $R_2$ are as defined in Claim 1